(19) Europäisches Patentamt
European Patent Office
Office européen des brevets

(11) EP 1 436 335 B1

(12) # EUROPÄISCHE PATENTSCHRIFT

(45) Veröffentlichungstag und Bekanntmachung des Hinweises auf die Patenterteilung:
**26.01.2005 Patentblatt 2005/04**

(21) Anmeldenummer: **02777242.5**

(22) Anmeldetag: **27.09.2002**

(51) Int Cl.[7]: **C08F 8/30**, A61L 15/00

(86) Internationale Anmeldenummer:
**PCT/EP2002/010866**

(87) Internationale Veröffentlichungsnummer:
**WO 2003/031482 (17.04.2003 Gazette 2003/16)**

(54) **VERFAHREN ZUR VERNETZUNG VON HYDROGELEN MIT MORPHOLIN-2,3-DIONEN**
METHOD FOR CROSSLINKING HYDROGELS WITH MORPHOLINE-2,3-DIONES
PROCEDE DE RETICULATION D'HYDROGELS CONTENANT DES MORPHOLINE-2,3-DIONES

(84) Benannte Vertragsstaaten:
**AT BE BG CH CY CZ DE DK EE ES FI FR GB GR IE IT LI LU MC NL PT SE SK TR**

(30) Priorität: **05.10.2001 DE 10149267**

(43) Veröffentlichungstag der Anmeldung:
**14.07.2004 Patentblatt 2004/29**

(73) Patentinhaber: **BASF AKTIENGESELLSCHAFT**
**67056 Ludwigshafen (DE)**

(72) Erfinder:
- **DANIEL, Thomas**
**67165 Waldsee (DE)**
- **EXNER, Kai, Michael**
**69214 Eppelheim (DE)**
- **MASSONNE, Klemens**
**67098 Bad Dürkheim (DE)**
- **RIEGEL, Ulrich**
**66849 Landstuhl (DE)**
- **WEISMANTEL, Matthias**
**63637 Jossgrund (DE)**

(56) Entgegenhaltungen:
**WO-A-00/31153**
**WO-A-99/42494**
**WO-A-99/43720**

- **HARWOOD L M ET AL: "A Novel Synthetic Route to Morpholin-2,3-Diones from 2-Aminoalcohols" TETRAHEDRON LETTERS, ELSEVIER SCIENCE PUBLISHERS, AMSTERDAM, NL, Bd. 37, Nr. 24, 10. Juni 1996 (1996-06-10), Seiten 4217-4220, XP004029120 ISSN: 0040-4039**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Gel- bzw. Oberflächennachvernetzung von wasserabsorbierenden Hydrogelen mit Morpholin-2,3-dionen und die nachvernetzten Hydrogele und deren Verwendung.

Quellbare Hydrogel-bildende Polymerisate, sogenannte Superabsorber (Super-Absorbing Polymers), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber weren außerdem in anderen Gebieten der Technik eingesetzt, bei denen Flüssigkeiten, insbesondere Wasser oder wässrige Lösungen absorbiert werden. Diese Gebiete sind z.B. Lagerung, Verpackung, Transport (Verpackungsmaterial wassersensitiver Artikel, wie z.B. Blumentransport, Schock Protection); Lebensmittelsektor (Transport von Fisch, Frischfleisch; Absorption von Wasser, Blut in Frischfisch/-Fleisch-Verpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textil (Handschuhe, Sportbekleidung, Feuchtigkeitsregulation in Textilien, Schuheinlagen); chem.-techn. Anwendungen (Katalysator für org. Reaktionen, Immobilisierung großer funktioneller Moleküle (Enzyme), Adhesiv für Agglomerationen, Wärmespeicher, Filtrationshilfsmittel, hydrophile Komponente in Polymerlaminaten, Dispergiermittel, Verflüssiger); Bau- und Konstruktionswesen, Installation (Pulverspritzguss, lehmbasierende Putze, Vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freitsetzung von Wirkstoffen an Pflanzen); im Feuerschutz (Funkenflug) (Abdecken von Häusern bzw. Bedecken von Hauswänden mit SAP Gel, da das Wasser eine sehr hohe Wärmekapazität hat, kann ein Entflammen verhindert werden; Versprühen von SAP Gel bei Bränden wie z.B. Waldbränden); Coextrustionsmittel in thermoplastischen Polymeren (Hydrophilierung von Mehrschicht)folien; Herstellung von Folien und thermoplastischen Formkörpern, die Wasser absorbieren können (z.B. Regen- und Tauwasser speichernde Folien für die Landwirtschaft; SAP haltige Folien zum Frischhalten von Obst und Gemüse, die in feuchte Folien verpackt werden können; der SAP speichert vom Obst und Gemüse abgegebenes Wasser ohne Bildung von Kondensationstropfen und gibt das Wasser teilweise an das Obst und Gemüse wieder ab, so daß weder Faulen noch Verwelken einritt; SAP-Polystyrol Coextrudate z.B. für Lebensmittelverpackungen wie Fleisch, Fisch, Geflügel, Obst und Gemüse); Trägersubstanz in Wirkstoffformulierungen (Pharma, Pflanzenschutz). In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass herkömmliche Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) darf das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden. Das Phänomen des Gelblokkings wird somit unterbunden, das im Extremfall zum Austritt der Flüssigkeit, der sog. Leakage des Hygieneartikels führt. Die Flüssigkeitsweiterleitung und -verteilung ist also bei der anfänglichen Absorption von Körperflüssigkeiten von entscheidender Bedeutung.

Gute Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Eine elegante Methode zur Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vernetzungsdichte in der Schale der Superabsorberpartikel, wodurch die Absorption unter Druckbelastung auf ein höheres

Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wäßrigen Flüssigkeiten quellbare Naturprodukte, wie beispielsweise Guarderivate. Solche Hydrogele werden als wäßrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Dazu geeignete Vernetzer sind Verbindungen, die mindestens zwei Gruppen enthalten, die mit den Carboxylgruppen des hydrophilen Polymeren kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werden können (siehe beispielsweise EP-A-0 083 022, EP-A-0 543303 und EP-A-0 530 438). Als Vernetzer geeignete Polyamidoamine sind insbesondere in EP-A-0 349 935 beschrieben.

Ein wesentlicher Nachteil dieser Vernetzer ist deren hohe Reaktivität, da diese besondere Schutzvorkehrungen im Produktionsbetrieb erforderlich macht, um unerwünschte Nebeneffekte zu vermeiden. Ebenso besitzen die vorgenannten Vernetzer hautreizende Eigenschaften, was bei der Verwendung in Hygieneartikeln problematisch erscheint.

Als Vernetzer sind auch polyfunktionelle Alkohole bekannt. Beispielsweise lehren EP-A-0 372 981, US-4 666 983 sowie US-5 385 983 die Verwendung von hydrophilen Polyalkoholen bzw. die Verwendung von Polyhydroxytensiden. Die Reaktion wird hiernach bei hohen Temperaturen von 120 - 250°C durchgeführt. Das Verfahren hat den Nachteil, dass die zur Vernetzung führende Veresterungsreaktion selbst bei diesen Temperaturen nur langsam abläuft.

Desweiteren sind als Vernetzer in WO 99/42494 auch 2-Oxazolidon und dessen Derivate, in WO 00/31153 2-Oxotetrahydro-1,3-oxazin und dessen Derivate, in WO 00/31152 N-Acyl-2-Oxazolidone und in WO 99/43720 Bis- und Poly-2-oxazolidinone als geeignete Vernetzer beschrieben. Diese erfüllen zwar die Anforderungen hinsichtlich einer Verwendung in Hygieneartikeln, sind aber als Verbindungen kommerziell nicht erhältlich und relativ schwierig in Reinform herstellbar.

Weiterhin sind als Vernetzer β-Hydroxyalkylamide in US 6239230 beschrieben. Auch diese sind gut geeignet für den Einsatz in Hygieneartikeln. Der Nachteil dieser Verbindungen liegt in den notwendigen relativ hohen Einsatzmengen und den damit zusammenhängenden Kosten.

Es bestand daher die Aufgabe, weitere Vernetzungssubstanzen für Superabsorber zu finden, die bei möglichst kurzer Reaktionszeit und möglichst tiefen Reaktionstemperaturen zu verwenden sind.

Überraschenderweise wurde nun gefunden, dass Morpholin-2,3-dione als Vernetzer hervorragend zur Lösung dieser Aufgabe geeignet sind.

Morpholin-2,3-dione an sich sind in der Literatur bekannt als Ausgangsstoffe zur Herstellung bestimmter Oxaminsäurederivate EP 371640.

Gegenstand der Erfindung ist Verfahren zur Vernetzung von Ausgangspolymeren zur Herstellung von wässrige Flüssigkeiten absorbierende Hydrogel-formende Polymere, charakterisiert durch die Verwendung eines Vernetzers der allgemeinen Formel 1:

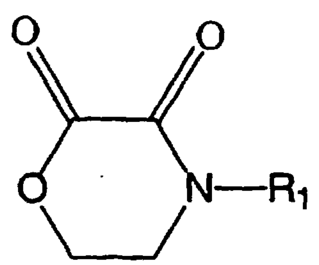

(Formel 1)

wobei der Rest $R_1$ entweder Wasserstoff, $C_1$-$C_4$-Alkyl, $C_1$-$C_4$-Hydroxyalkyl oder eine Gruppe der Formel 2 darstellt:

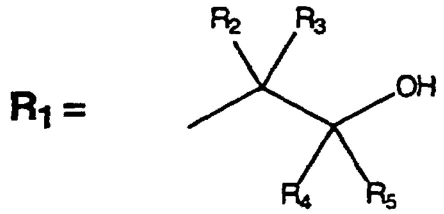

(Formel 2)

worin $R_2$, $R_3$, $R_4$, $R_5$ voneinander unabhängig sind und $C_1$-$C_4$-Alkyl, $C_1$-$C_4$-Hydroxyalkyl, oder Wasserstoff bedeuten. Unter Vernetzung wird sowohl die Gelvernetzung (Quervernetzung von linearem oder schwach vernetzten Polymer) als auch die Oberflächennachvernetzung verstanden. Durch Oberflächennachvernetzung wird die oberflächlich stärkere Quervernetzung des Ausgangspolymeren verstanden, die zu einer Kern-Schale Struktur führt. Unter Ausgangspolymer wird das Polymer vor der Vernetzung verstanden. Wässrige Flüssigkeiten absorbierende Hydrogel-formende Polymere sind bevorzugt solche mit einer Absorption von destilliertem Wasser von mindestens dem Eigengewicht, bevorzugt dem 10-fachem Eigengewicht, diese Absorption wird bevorzugt auch unter einem Druck von 0.7 psi erreicht.

Die Oberflächennachvernetzung ist bevorzugt. Das Ausgangspolymer enthält bevorzugt Carboxylgruppen.

Bei den oben beschriebenen Verfahren zur Vernetzung wird das Ausgangspolymer mit einem Vernetzer behandelt und bevorzugt während oder nach dem Behandeln durch Temperaturerhöhung nachvernetzt und getrocknet wird, wobei der Vernetzer bevorzugt in einem inerten Lösemittel enthalten ist. Unter inerten Lösemittel werden solche verstanden, die bei der Reaktion weder mit dem Ausgangspolymer noch mit dem Vernetzer im Wesentlichen reagieren. Bevorzugt sind solche Lösemittel, die zu mehr als 90%, bevorzugt mehr als 95%, besonders bevorzugt mehr als 99%, insbesondere zu mehr als 99,5% nicht chemisch mit dem Ausgangspolymer oder Vernetzer reagieren.

Einen besonders bevorzugten Vernetzer in den obigen Verfahren stellt das N-2-Hydroxyethyl-Morpholin-2,3-dion (Formel 3) dar:

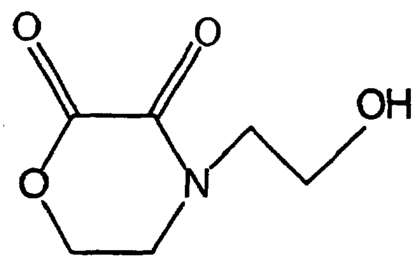

(Formel 3)

Dieses ist besonders einfach und kostengünstig herstellbar.

Bevorzugt zur Nachvernetzung und Trocknung ist dabei der Temperaturbereich zwischen 30 und 250°C, insbesondere 50 - 200°C, ganz besonders bevorzugt ist der Bereich zwischen 100 - 180°C. Die Aufbringung der Oberflächennachvernetzungslösung erfolgt bevorzugt durch Aufsprühen auf das Polymere in geeigneten Sprühmischern. Im Anschluß an das Aufsprühen wird das Polymerpulver thermisch getrocknet, wobei die vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX-Mischer, NAUTA-Mischer, SHUGGI-Mischer oder PROCES-SALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch z.B. eine azeotrope Destillation als Trocknungsverfahren benutzt werden. Die bevorzugte Verweilzeit bei dieser Temperatur im Reaktionsmischer oder Trockner beträgt unter 60 min., besonders bevorzugt unter 30 min.

Bevorzugt sind obige Verfahren, wobei das Ausgangspolymere eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Die Erfindung betrifft außerdem ein Stoffgemisch enthaltend einen Vernetzer der Formel 1 oder den bevorzugten Vernetzer und Lösemittel ausgewählt aus der Gruppe bestehend aus Wasser, ein Gemisch von Wasser mit einem oder mehreren in Wasser unbegrenzt löslichen organischen Lösemittel oder ein Gemisch von Wasser mit einem

oder mehreren einfachen oder mehrfachfunktionellen Alkoholen, z.B. Methanol und Glycerin. Die Alkohole tragen bevorzugt 1, 2 oder 3 OH Gruppen und weisen bevorzugt zwischen 1 bis 10, insbesondere bis 4 C-Atome auf. Bevorzugt sind primäre und sekundäre Alkohole.

Bei dem Stoffgemisch liegt bevorzugt das Lösemittel in einem Alkohol/Wasser-Gemisch mit einem Alkoholgehalt dieser Lösung von 10 - 90 Gew.-%, bevorzugt 30 - 70 Gew.-%, mehr bevorzugt 15 - 65 Gew.-%, insbesondere 40 - 60 Gew.-% vor.

Bevorzugte Alkohole sind Methanol, Ethanol, Isopropanol, Ethylenglykol, 1,2-Propandiol oder 1,3-Propandiol.

Bevorzugtes Stoffgemisch enthält an Vernetzer im Stoffgemisch 0.1 bis 20 Gew.-%, insbesondere 0.5 bis 10, bevorzugt bis 3 Gew.-%.

In bevorzugten obigen Verfahren wird ein oben genanntes Stoffgemisch verwendet.

Bevorzugt werden solche Verfahren in denen das Stoffgemisch enthaltend Vernetzer und Lösemittel in einem Verhältnis von 0,1 - 20 Gew.-%, insbesondere 0,5 - 10 Gew.-% bezogen auf die Masse des Ausgangspolymeren eingesetzt wird.

Bevorzugt werden solche Verfahren in denen der Vernetzer in einer Dosierung von 0,01 - 5,0 Gew.%, bevorzugt 0,02 - 3,0 Gew.%, ganz besonders bevorzugt 0,03 - 1,0 Gew.%, insbesondere 0,05 - 0,1 Gew.% bezogen auf das Ausgangspolymer verwendet wird.

Beansprucht werden auch Polymere hergestellt nach einem der oben genannten Verfahren und deren Verwendung in Hygieneartikeln, Verpackungsmaterialien und in Nonwovens, sowie die Verwendung von einem oben genannten Stoffgemisch zur Herstellung von vernetzten oder durch Wärmebehandlung vernetzungsfähigen Polymeren, insbesondere in Lacken und Farben.

Die im erfindungsgemäßen Verfahren einzusetzenden hydrophilen, hochquellfähigen Hydrogele (Ausgangspolymere) sind insbesondere Polymere aus (co)polymerisierten hydrophilen Monomeren, Pfropf(co)polymere von einem oder mehreren hydrophilen Monomeren auf eine geeignete Pfropfgrundlage, vernetzte Cellulose- oder Stärkeether oder in wäßrigen Flüssigkeiten quellbare Naturprodukte, wie beispielsweise Guarderivate. Diese Hydrogele sind dem Fachmann bekannt und beispielsweise beschrieben in US-4 286 082, DE-C-27 06 135, US-4 340 706, DE-C-37 13 601, DE-C-28 40 010, DE-A-43 44 548, DE-A40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780 EP-A-0 20 5674, US-5 145 906, EP-A-0 530 438, EP-A-0 670 073, US4 057 521, US-4 062 817, US-4 525 527, US-4 295 987, US-5 011 892, US-4 076 663 oder US-4 931 497. Besonders geeignet sind auch hochquellfähige Hydrogele aus einem Herstellprozeß wie in WO 01/38402 beschrieben, sowie anorganisch-organische hybride hochquellfähige Hydrogele wie in DE 198 54 575 beschrieben. Der Inhalt der vorstehend genannten Patentdokumente, insbesondere die nach den Verfahren hergestellten Hydrogele, sind ausdrücklich Bestandteil der vorliegenden Offenbarung.

Zur Herstellung dieser hydrophilen, hochquellfähigen Hydrogele geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2methylpropansulfonsäure, 2-Acrylamido-2-methylpropanphosphonsäure sowie deren Amide, Hydroxyalkylester und aminogruppen- oder ammoniumgruppenhaltige Ester und Amide. Desweiteren wasserlösliche N-Vinylamide oder auch Diallyldimethyl-ammoniumchlorid. Bevorzugte hydrophile Monomere sind Verbindungen der Formel 4

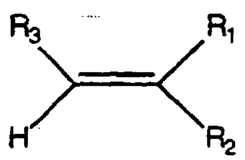

(Formel 4)

worin

$R_1$ Wasserstoff, Methyl oder Ethyl,

$R_2$ die Gruppe $-COOR_4$, eine Sulfonylgruppe oder Phosphonylgruppe, eine mit einem $(C_1-C_4)$-Alkanol veresterte Phosphonylgruppe oder eine Gruppe der Formel 5

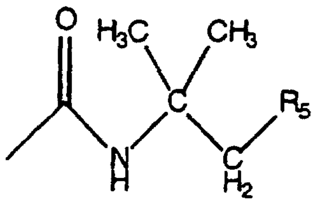

(Formel 5)

$R_3$ Wasserstoff, Methyl, Ethyl oder eine Carboxylgruppe,
$R_4$ Wasserstoff, Amino oder Hydroxy-($C_1$-$C_4$)-Alkyl und
$R_5$ eine Sulfonylgruppe, eine Phosphonylgruppe oder eine Carboxylgruppe bedeuten.

Beispiele für ($C_1$-$C_4$)-Alkanole sind Methanol, Ethanol, n-Propanol oder n-Butanol.

Besonders bevorzugte hydrophile Monomere sind Acrylsäure und Methacrylsäure.

Geeignete Pfropfgrundlagen für hydrophile Hydrogele, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das wasserabsorbierende Polymer kann über radikalische Pfropfcopolymerisation von Acryl-säure oder Acrylat auf eine wasserlösliche Polymermatrix erhalten werden. Geeignete wasserlösliche Polymermatrices sind beispielsweise, aber nicht ausschliesslich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikal-vernetzer eingesetzt.

Das wasserabsorbierende Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozeß ein in Wasser lösliches Silikat oder lösliches Aluminat oder Gemische von beiden eingesetzt wurde.

Geeignete Polyalkylenoxide haben beispielsweise die Formel 6

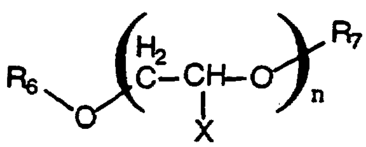

(Formel 6)

worin

$R_6$ und $R_7$ unabhängig voneinander Wasserstoff, Alkyl, Alkenyl oder Acryl,
X Wasserstoff oder Methyl und
n eine ganze Zahl von 1 bis 10 000 bedeuten.

$R_6$ und $R_7$ bedeuten bevorzugt Wasserstoff, ($C_1$-$C_4$)-Alkyl, ($C_2$-$C_6$)-Alkenyl oder Phenyl.

Bevorzugte Hydrogele sind insbesondere Polyacrylate, Polymethacrylate sowie die in US-4 931 497, US-5 011 892 und US-5 041 496 beschriebene Pfropfpolymere. Ganz besonders bevorzugte Hydrogele sind die in WO 01/38402 beschriebenen Kneterpolymere und die in DE 198 545 75 beschriebenen hybriden organisch-anorganischen Hydrogele auf Basis von Polyacrylaten.

Die hydrophilen, hochquellfähigen Hydrogele sind bevorzugt vernetzt, d. h. sie enthalten Verbindungen mit mindestens zwei Doppelbindungen, die in das Polymernetzwerk einpolymerisiert sind. Geeignete Vernetzer sind insbesondere Methylenbisacryl- bzw. - methacrylamid, Ester ungesättigter Mono- oder Polycarbonsäuren von Polyolen, wie Diacrylat oder Triacrylat, z. B. Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Besonders bevorzugt werden im erfindungsgemäßen Verfahren jedoch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Monoethylenglykol-diallylether, Glyceroldi- und Triallylether, Polyallylether auf Basis Sorbitol, sowie ethoxylierte Varianten davon. Weiterhin besonders bevorzugte Vernetzer sind die Polyethylenglykol-diacrylate, ethoxylierte Derivate von Trimethylolpropantriacrylat z.B. Sartomer SR 9035, sowie ethoxylierte Derivate von Glycerindiacrylat und Glycerintriacrylat. Selbstverständlich können auch Gemische der obigen Vernetzer eingesetzt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001 - 10 mol-%), ganz besonders bevorzugt sind jedoch.Polymere, die über radikalische Polymerisation erhalten wurden und bei denen ein mehrfunktioneller ethylenisch ungesättigter Radikalvernetzer verwendet wurde.

Die hydrophilen, hochquellfähigen Hydrogele können durch an sich bekannte Polymerisations-verfahren hergestellt werden. Bevorzugt ist die Polymerisation in wäßriger Lösung nach dem Verfahren der sogenannten Gelpolymerisation. Dabei werden 15 bis 50 Gew.-% ige wäßrige Lösungen eines oder mehrerer hydrophiler Monomerer und gegebenenfalls einer geeigneten Pfropfgrundlage in Gegenwart eines Radikalinitiators bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren herangezogen werden, z. B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$. $K_2S_2O_8$ oder $H_2O_2$.

Sie können gegebenenfalls in Kombination mit Reduktionsmitteln wie z.B. Ascorbinsäure, Natriumhydrogensulfit, und Eisen(11)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie z. B. Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich 50º bis 130°C, vorzugsweise 70º bis 100°C, können die Qualitätseigenschaften der Polymerisate noch verbessert werden.

Die erhaltenen Gele werden zu 0 - 100 mol-%, bevorzugt zu 25 - 100 mol-%, und besonders bevorzugt zu 50 - 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat.

Üblicherweise wird die Neutralisation durch Einmischung des Neutralisationsmittels als wäßrige Lösung oder bevorzugt auch als Feststoff erreicht. Das Gel wird hierzu mechanisch zerkleinert, z.B. mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels UV-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

Das getrocknete Hydrogel wird hiernach gemahlen und gesiebt, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 - 1000 µm, bevorzugt bei 45 - 850 µm, besonders bevorzugt bei 200 - 850 µm, und ganz besonders bevorzugt bei 300 - 850 µm. In diesen Bereichen liegen bevorzugt 80 Gew.-% der Teilchen, insbesondere 90 Gew.-% der Teilchen. Die Größenverteilung kann mit etablierten Lasermethoden bestimmt werden.

Der CRC-Wert [g/g] der erfindungsgemäßen Hydrogel-formenden Polymere kann nach den in der Beschreibung angegebenen Methoden gemessen werden und liegt bevorzugt größer 15, insbesondere 16, 18, 20 22, 24, oder höher, besonders bevorzugt bei 25 insbesondere bei 26, 27, 28, 29, insbesondere bevorzugt bei 30, 31, 32, 33, 34, 35, 36, 37 oder höher.

Der AUL-0.7psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Der AUL-0.5psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Einsatz und Verwendung erfindungsgemäßen Hydrogel-formender Polymerer

Die vorliegende Erfindung betrifft ferner die Verwendung der oben genannten Hydrogel-formenden Polymeren in Hygieneartikel, umfassend

(A) eine obere flüssigkeitsdurchlässige Abdeckung
(B) eine untere flüssigkeitsundurchlässige Schicht
(C) einen zwischen (A) und (B) befindlichen Kern, enthaltend 10 - 100 Gew.-% des erfindungsgemäßem Hydrogel-formenden Polymer
0 - 90 Gew.-% hydrophiles Fasermaterial
bevorzugt 20 - 100 Gew.-% des erfindungsgemäßem Hydrogel-formenden Polymer, 0 - 80 Gew.-% hydrophiles Fasermaterial
mehr bevorzugt 30 - 100 Gew.-% des erfindungsgemäßem Hydrogel-formenden Polymer, 0 - 70 Gew.-% hydrophiles Fasermaterial
noch mehr bevorzugt 40 - 100 Gew.-% des erfindungsgemäßem Hydrogel-formenden Polymer, 0 - 60 Gew.-% hydrophiles Fasermaterial
besser bevorzugt 50 - 100 Gew.-% des erfindungsgemäßem Hydrogel-formenden Polymer 0 - 50 Gew.-% hydrophiles Fasermaterial
besonders bevorzugt 60 - 100 Gew.-% des erfindungsgemäßem Hydrogel-formenden Polymer, 0 - 40 Gew.-% hydrophiles Fasermaterial
insbesondere bevorzugt 70 - 100 Gew.-% des erfindungsgemäßem Hydrogel-formenden Polymer, 0 - 30 Gew.-% hydrophiles Fasermaterial
außerordentlich bevorzugt 80 - 100 Gew.-% des erfindungsgemäßem Hydrogel-formenden Polymer, 0 - 20 Gew.-% hydrophiles Fasermaterial
am meisten bevorzugt 90 - 100 Gew.-% des erfindungsgemäßem Hydrogel-formenden Polymer, 0 - 10 Gew.-% hydrophiles Fasermaterial
(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Die Prozentangaben sind so zu verstehen, dass bei 10 - 100 Gew.%, 11, 12, 13, 14, 15, 16, 17, 18, 19 bis jeweils 100 Gew.-% an erfindungsgemäßem Hydrogel-formenden Polymer und alle dazwischenliegendnen %-Angaben (z.B. 12,2%) möglich sind und entsprechend hydrophiles Fasermaterial von 0 bis jeweils 89, 88, 87, 86, 85, 83, 82, 81 Gew.-% und dazwischen liegende Prozentangaben (z.B. 87,8%) möglich sind. Liegen weitere Materialien im Kern vor, so verringern sich entsprechend die Prozentwerte an Polymer und Fäser. Das ananloge gilt für die bevorzugten Bereiche, so z.B. bei außerordentlich bevorzugt können 81, 82, 83, 84, 85, 86, 87, 88, 89 Gew.% für das erfindungsgemäßem Hydrogel-formenden Polymer und entsprechend 19, 18, 17, 16, 15, 14, 13, 12, 11 Gew-% des Fasermaterials vorliegen. So können im bevorzugten Bereich 20, 21, 22, 23, 24, 25, 26, 27, 28, 29 bis 100 Gew.-% erfindungsgemäßes Hydrogel-formendes Polymer, im mehr bevorzugten Bereich 30, 31, 32, 33, 34, 35, 36, 37, 38, 39 bis 100 Gew.-% erfindungsgemäßes Hydrogel-formendes Polymer, im noch mehr bevorzugten Bereich 40, 41, 42, 43, 44, 45, 46, 47, 48, 49 bis 100 Gew.-% erfindungsgemäßes Hydrogel-formendes Polymer, im besser bevorzugten Bereich 50, 51, 52, 53, 54, 55, 56, 57, 58, 59 bis 100 Gew.-% erfindungsgemäßes Hydrogel-formendes Polymer, im besonders bevorzugten Bereich 60, 61, 62, 63, 64, 65, 66, 67, 68, 69 bis 100 Gew.-% erfindungsgemäßes Hydrogel-formendes Polymer, im insbesonders bevorzugten Bereich 70, 71, 71, 72, 73, 74, 75, 76, 77, 78, 79 bis 100 Gew.-% erfindungsgemäßes Hydrogel-formendes Polymer und im am meisten bevorzugten Bereich 90, 91, 92, 93, 94, 95, 96, 97, 98, 99 oder 100 Gew.-% erfindungsgemäßes Hydrogel-formendes Polymer vorliegen.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 102 388 3 A2.

Die flüssigkeitsundurchlässige Schicht (B) besteht in der Regel aus einer Folie aus Polyethylen oder Polypropylen.

Der Kern (C) enthält neben derm erfindungsgemäßen Hydrogel-formendem Polymer hydrophiles

Fasermaterial . Unter hydrophil ist zu verstehen, daß sich wäßrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 - 200 µm, bevorzugt 10 - 100 µm. Darüberhinaus haben die Fasern eine Mindestlänge von 1 mm.

Der Aufbau und die Form von Windeln ist allgemein bekannt und beispielsweise in der WO 95 / 26 209 S. 66 Zeile 34 bis S. 69 Zeile 11, DE 196 04 601 A1, EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 - 17, WO 00/35502, insbesondere Seiten 3-9, DE 19737434, WO 98/8439 beschrieben. Hygieneartikel für die Damenhygiene werden in folgenden Literaturstellen beschrieben. Die erfindungsgemäßen wäßrige Flüssigkeiten absorbierende Hydrogel-formende Polymere können dort eingestzt werden. Literaturstellen Damenhygiene: WO 95/24173: Absorption Article for Controlling Odour, WO 91/11977: Body Fluid Odour Control, EP 389023: Absorbent Sanitary Articles, WO 94/25077: Odour Control Material, WO 97/01317: Absorbent Hygienic Article, WO 99/18905, EP 834297, US 5,762,644, US 5,895,381, WO 98/57609, WO 2000/065083, WO 2000/069485, WO 2000/069484, WO 2000/069481, US 6,123,693, EP 1104666, WO 2001/024755, WO 2001/000115, EP 105373, WO 2001/041692, EP 1074233. Tampons werden in folgenden Schriften beschrieben: WO 98/48753, WO 98/41179, WO 97/09022, WO 98/46182, WO 98/46181, WO 2001/043679, WO 2001/043680, WO 2000/061052, EP 1108408, WO 2001/033962, DE 200020662, WO 2001/001910, WO 2001/001908, WO 2001/001909, WO 2001/001906, WO 2001/001905, WO 2001/24729. Inkontinenzartikel werden in folgenden Schriften beschrieben: Disposable Absorbent Article for Incontinent Individuals: EP 311344 Beschreibung S. 3 - 9; Disposable Absorbent Article: EP 850623; Absorbent Article: WO 95/26207; Absorbent Article: EP 894502; Dry Laid Fibrous Structure: EP 850 616; WO 98/22063; WO 97/49365; EP 903134; EP 887060; EP 887059; EP 887058; EP 887057; EP 887056; EP 931530; WO 99/25284; WO 98/48753. Damenhygiene- und Inkontinenzartikel wird in folgenden Schriften beschrieben: Catamenial Device: WO 93/22998 Beschreibung S. 26 - 33; Absorbent Members for Body Fluids: WO 95/26209 Beschreibung S. 36 - 69; Disposable Absorbent Article: WO 98/20916 Beschreibung S. 13 - 24; Improved Composite Absorbent Structures: EP 306262 Beschreibung S. 3 - 14; Body Waste Absorbent Article: WO 99/45973. Diese Referenzen und die Referenzen dort, werden hiermit ausdrücklich in die Offenbarung der Erfindung einbezogen.

Die erfindungsgemäßen Hydrogel-formenden Polymere eignen sich hervorragend als Absorptionsmittel für Wasser und wäßrige Flüssigkeiten, so daß sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Einlagerung und Fixierung der erfindungsgemäßen hochquellfähigen Hydrogele

Zusätzlich zu den oben beschriebenen hochquellfähigen Hydrogelen liegen in der absorbierenden Zusammensetzung gemäß der vorliegenden Erfindung Kompositionen vor, welche die hochquellfähigen Hydrogele enthalten oder an denen sie fixiert sind. Jede Komposition ist geeignet, die die hochquellfähigen Hydrogele aufnehmen kann und die darüber hinaus in die Absorptionsschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (air-laid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines air-laid web ist beispielsweise geschildert in WO 98/28 478. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. Ein derartiges Kammersystem ist detailliert geschildert in EP 0 615 736 A1 S. 7 Zeile 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die hochquellfähigen Hydrogelpartikel zurückzuhalten. Obige Beispiele zur Komposition der absorbierenden Zusammensetzung schließen auch Laminate aus mindestens zwei Schichten mit ein, zwischen die die hochquellfähigen Hydrogele eingebaut und fixiert werden.

Generell besteht die Möglichkeit, Hydrogelpartikel innerhalb des Absorbent Cores zur Verbesserung der sog. Dry- und Wet-Integrity zu fixieren. Unter Dry- und Wet-Integrity versteht man die Fähigkeit, hochquellfähige Hydrogele derart in die absorbierende Zusammensetzung einzubauen, daß sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem

im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in WO 95/26 209 S. 37 Zeile 36 bis S. 41 Zeile 14. Besagte Passage ist somit Bestandteil dieser Erfindung. Methoden zur Erhöhung der Wet Strength finden sich auch in WO 2000/36216 A1.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die hochquellfähigen Hydrogele mit einem Gewichtsanteil von 10 bis 100 Gew%, bevorzugt 20 - 100 Gew.-%, mehr bevorzugt 30 - 100 Gew.-%, noch mehr bevorzugt 40 - 100 Gew.-%, besser bevorzugt 50 - 100 Gew.-%, besonders bevorzugt 60 - 100 Gew.-%, insbesondere bevorzugt 70 - 100 Gew.-%, außerordentlich bevorzugt 80 - 100 Gew.-% und am meisten bevorzugt 90 - 100 Gew.-% basierend auf dem Gesamtgewicht der Komposition und der hochquellfähigen Hydrogele eingebaut.

Fasermaterialien der absorbierenden Zusammensetzung

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt Patent WO 95/26 209 S. 28 Zeile 9 bis S. 36 Zeile 8. Besagte Passage ist somit Bestandteil dieser Erfindung.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DACRON® oder KO-DEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75°C und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0.1 bis 100 Denier (Gramm pro 9 000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der

Kontaktwinkel zwischen der Flüssigkeit und der Faser (bzw. ihrer Oberfläche) kleiner aus 90º ist, oder wenn die Flüssigkeit zum spontanen Spreiten auf derselben Oberfläche tendiert.

Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikel werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser : (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Hercoles, Inc. Wilmington Delaware, USA), Polyacrylamid- Überzüge (beschrieben in U.S. Patent 3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielsweise Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, U.S. Patent 3,224,926, U.S. Patent 3,440,135, U.S. Patent 3,932,209, U.S. Patent 4,035,147, U.S. Patent 4,822,453, U.S. Patent 4,888,093, U.S. Patent 4,898,642 und U.S. Patent 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Herstellungsverfahren der absorbierenden Zusammensetzung

Die absorbierende Zusammensetzung setzt sich zusammen aus Kompositionen, welche hochquellfähige Hydrogele enthalten, und den hochquellfähigen Hydrogelen, die in besagten Kompositionen vorliegen oder daran fixiert sind.

Beispiele für Verfahren, mit denen man eine absorbierende Zusammensetzung erhält, die beispielsweise aus einem Trägermaterial bestehen, an den ein- oder beidseitig hochquellfähige Hydrogele fixiert sind, sind bekannt und von der Erfindung mit eingeschlossen, jedoch nicht limitiert darauf.

Beispiele für Verfahren, mit denen man eine absorbierende Zusammensetzng erhält, die beispielsweise aus in ein Fasermaterial-Gemisch aus synthetischen Fasern (a) und Cellulosefasern (b) eingebetteten hochquellfähigen Hydrogelen (c) besteht, wobei das Mischungsverhältnis von (100 bis 0) synthetische Faser : (0 bis 100) Cellulosefaser

variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so daß eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0.5 Sekunde bis 3 Minuten, bevorzugt 1 Sekunde bis 1 Minute.

Die absorbierende Zusammensetzung wird im allgemeinen beispielsweise mit einer für Flüssigkeit durchlässien Deckschicht und einer für Flüssigkeit undurchlässigen Unterschicht versehen. Wieterhin werden Beinabschlüsse und Klebebänder angebracht und so der Hygieneartikel fertiggestellt. Die Materialien und Arten der durchlässien Deckschicht und undurchlässigen Unterschicht, sowie der Beinabschlüsse und Klebebänder sind dem Fachmann bekannt und nicht besonders eingeschränkt. Beispiele hierfür finden sich in WO 95/26 209.

Experimenteller Teil

Zur Bestimmung der Güte der Oberflächenvernetzung kann das getrocknete Hydrogel mit folgenden Testmethoden untersucht werden.

Testmethoden

a) Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0.2000 ± 0.0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0.9 Gew.%igen Kochsalzlösung gegeben (mindestens 0,83 l KochsalzLösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

b) Absorption unter Druck (AUL Absorbency Under Load) (0.7 psi)

Die Messzelle zur Bestimmung der AUL 0.7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1345 g. Zur Durchführung der Bestimmung der AUL 0.7 psi wird das Gewicht des leeren Plexiglas-Zylinders und der Plastikplatte ermittelt und als $W_0$ notiert. Dann werden 0,900 ± 0.005 g Hydrogel-formendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0.9 Gew.%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der

Plastikplatte ausgewogen und das Gewicht als $W_b$ notiert.

Die Absorption unter Druck (AUL) wird wie folgt berechnet:

$$AUL\ 0.7\ psi\ [g/g] = [W_b\text{-}W_a] / [W_a\text{-}W_0]$$

Der AUL 0.5psi wird analog mit geringerem Druck gemessen.

c) Saline Flow Conductivity (SFC)

Die Testmethode zur Bestimmung der SFC ist beschrieben in U.S. 5 599 335.

Beispiele

Herstellbeispiel für N-2-Hydroxyethyl-Morpholin-2,3-dion:

657.6 g (4.5 mol) Oxalsäurediethylester werden in einem 2 l Vierhalskolben mit Rückflußkühler, Thermometer, KPG-Rührer und Tropftrichter vorgelegt und auf 80°C aufgeheizt.

Im Laufe von 2 h werden 473.1 g (4.5 mol) Diethanolamin zugetropft. Man rührt 2 h bei 80°C nach und entfernt das freigesetzte Ethanol i. Vak., wobei ca. 749.4 g nahezu farbloser Rückstand erhalten werden, die noch Spuren von Ethanol enthalten.

Zur weiteren Reinigung werden 717.4 g des Rohproduktes in 1.8 l Ethanol in der Siedehitze gelöst. Nach Abkühlen auf Raumtemperatur wird das Kristallisat über einen Büchnertrichter abgesaugt und zweimal mit je 200 ml eiskaltem Ethanol nachgewaschen. Nach Trockenen i. Vak. Verbleiben 502.4 g (3.15 mol) farbloses, analysenreines Material.

Schmp. : 86.5°C (EtOH)

IR: 3400, 1757, 1682, 1148, 1055 cm-1

| | | | | | |
|---|---|---|---|---|---|
| Elementaranalyse | theoret | C: 45.31 | H: 5.7 | N: 8.8 | O: 40.2 |
| | gefunden | C: 45.1 | H: 5.7 | N: 8.8 | O: 40.0 |

Die Identität wird durch $^{1}H$- und $^{13}C$-NMR sowie Massenspektrum bestätigt.

Die anderen Morpholin-2,3-dion-Derivate können in analoger Weise oder durch Literatur-bekannte Verfahren hergestellt werden.

Hydrogel-Herstellbeispiel 1:

In einem 40 1-Plastikeimer werden 6,9 kg reine Acrylsäure verdünnt mit 23 kg Wasser, in dem 213 g Stärke gelöst sind (Paselli SA 2 der Firma AVEBE - Netherlands). Zu dieser Lösung fügt man 45 g Pentaerythritoltriallylether unter Rühren hinzu und inertisiert den verschlossenen Eimer durch Durchleiten von Stickstoff. Die Polymerisation wird dann durch Zugabe von ca. 400 mg Wasserstoffperoxid und 200 mg Ascorbinsäure gestartet. Nach Beendigung der Reaktion wird das Gel mechanisch zerkleinert und mit soviel Natronlauge versetzt bis ein Neutralisationsgrad von 75 mol-%, bezogen auf die eingesetzte Acrylsäure, erreicht wird (192 g NaOH 50 % pro kg Gel). Das neutralisierte Gel wird dann auf einem Walzentrockner getrocknet, mit einer Stiftmühle gemahlen und schließlich bei 300-800 Mikrometer abgesiebt.

Hydrogel-Herstellbeispiel 2:

In eine zur Inertisierung in einen PE-Beutel eingebunden rechtekkige Kunststoffschale einer Grundfläche von ca. 30x40 cm wird eine in einem separaten Gefäß hergestellte und bereits mit Stickstoff inertisierte Lösung über ein Schlauchsystem überführt. Die Lösung setzt sich aus folgenden Komponenten zusammen: 1466 g Wasser, 305 g Acrylsäure, 3204 g Natriumacrylat 37 %ig wässrig, 11 g SARTOMER SR 9035 (ethoxiliertes Trimethylolpropantriacrylat der Firma SARTOMER - USA), 0,61 g 2,2-Azobis-amidinopropan-dihydrochlorid und 3,1 g Natriumpersulfat. Zeitgleich zum Eintrag der Monomerlösung werden über 2 getrennte Schlauchsysteme 2 weitere Initiatorenlösungen so zudosiert,

dass eine homogene Durchmischung mit der Monomerlösung stattfindet. Es sind dies zum einen eine Lösung aus 0,25 g Wasserstoffperoxid in 5 g Wasser und zum anderen 0,25 g Ascorbinsäure in 5 g Wasser. Infolge Polymerisationsreaktion erhält man einen ca. 4cm dicken Gelquarder, der mechanisch mittels Fleischwolf zerkleinert, bei 160 °C im Umlufttrockenschrank getrocknet, mit einer Ultrazentrifugalmühle gemahlen und anschließend bei 300-850 µm abgesiebt.

Hydrogel-Herstellbeispiel 3:

Vorgehensweise und Zusammensetzung der Lösungen vollkommen analog zu Hydrogel-Herstellbeispiel-2. Der einzige Unterschied besteht darin, dass die Monomerlösung zusätzlich 0,5 g N-2-Hydroxyethylmorpholindion-2,3 enthält.

Beispiel 1:

20 g des Grundpolymers aus Hydrogel-Herstellbeispiel 1 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-2-Hydroxyethylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Vergleichsbeispiel 1:

20 g des Grundpolymers aus Hydrogel-Herstellbeispiel 1 wird in einem Waring-Labormischer mit Vernetzer-Lösung folgender Zusammensetzung besprüht: 1,5 Gew.-% Isopropanol, 3,5 Gew.-% Wasser, aber ohne jeden Zusatz von N-2-Hydroxyethylmorpholindion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Beispiel 2:

20 g des Grundpolymers aus Hydrogel-Herstellbeispiel 2 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-2-Hydroxyethylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Beispiel 3:

20 g des Grundpolymers aus Hydrogel-Herstellbeispiel 2 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-Methylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Beispiel 4:

20 g des Grundpolymers aus Hydrogel-Herstellbeispiel 2 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-Tert.-Butylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Beispiel 5:

20 g des Grundpolymers aus Hydrogel-Herstellbeispiel 2 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-Ethylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt,

um Klumpen zu entfernen.

Vergleichsbeispiel 2:

20 g des Grundpolymers aus Hydrogel-Herstellbeispiel 2 wird in einem Waring-Labormischer mit Vernetzer-Lösung folgender Zusammensetzung besprüht: 1,5 Gew.-% Isopropanol, 3,5 Gew.-% Wasser, aber ohne jeden Zusatz von Morpholin-dion-2,3-derivaten, wie sie in den Beispielen 2 bis 5 zum Einsatz gelangen, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Beispiel 6:

20 g des Grundpolymers aus Hydrogel-Herstellbeispiel 3 wird in einem Waring-Labormischer mit Vernetzer-Lösung folgender Zusammensetzung besprüht: 1,5 Gew.-% Isopropanol, 3,5 Gew.-% Wasser, 0,08 Gew.-% N-2-Hydroxyethylmorpholin-dion-2,3, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Vergleichsbeispiel 3:

20 g des Grundpolymers aus Hydrogel-Herstellbeispiel 3 wird in einem Waring-Labormischer mit Vernetzer-Lösung folgender Zusammensetzung besprüht: 1,5 Gew.-% Isopropanol, 3,5 Gew.-% Wasser, aber ohne jeden Zusatz von Morpholin-dion-2,3-derivaten, wie sie in dem Beispiel 6 zum Einsatz gelangen, jeweils bezogen auf eingesetztes Polymer. Anschließend wird das feuchte Produkt bei 175°C für 60 Minuten im Umlufttrockenschrank getempert. Das getrocknete Produkt wird anschließend bei 850 µm abgesiebt, um Klumpen zu entfernen.

Tabelle

| | Grundpolymer gemäß Hydro-gel-Herstellbei-spiel | Vernetzer | CRC (g/g) | AUL 0.5 psi (g/g) | AUL 0.7 psi (g/g) |
|---|---|---|---|---|---|
| Beispiel 1 | 1 | 0,08 % N-2-Hydroxyethyl-Morpholin-2,3-dion | 34,2 | 26,8 | 23,9 |
| Ver-gleichs-Beispiel 1 | 1 | — — | 38,3 | 9,4 | 8,4 |
| | | | | | |
| Beispiel 2 | 2 | 0,08 % N-2-Hydroxyethyl-Morpholin-2,3-dion | 35,6 | 25,2 | 19,5 |
| Beispiel 3 | 2 | 0,08 % N-Methyl-Morpho-lin-2,3-dion | 37,4 | 13,7 | 9,7 |
| Beispiel 4 | 2 | 0,08 % N-Tert.-Butyl-Mor-pholin-2,3-dion | 37,2 | 13,2 | 8,9 |
| Beispiel 5 | 2 | 0,08 % N-Ethyl-Morpho-lin-2,3-dion | 37,4 | 13,2 | 12,9 |
| Ver-gleichs-Beispiel 2 | 2 | — — | 39,7 | 7,9 | 6,5 |
| | | | | | |
| Beispiel 6 | 3 | 0,08 % N-2-Hydroxyethyl-Morpholin-2,3-dion | 34,0 | 27,6 | 24,2 |
| Ver-gleichs-Beispiel 3 | 3 | — — | 36,5 | 12,1 | 8,5 |

**Patentansprüche**

1. Verfahren zur Vernetzung von Ausgangspolymeren zur Herstellung von wässrige Flüssigkeiten absorbierende Hydrogel-formende Polymere, charakterisiert durch die Verwendung eines Vernetzers der allgemeinen Formel 1:

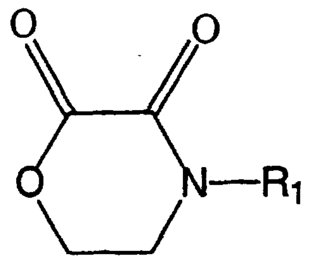

(Formel 1)

wobei der Rest $R_1$ entweder Wasserstoff, $C_1$-$C_4$-Alkyl, $C_1$-$C_4$-Hydroxyalkyl oder eine Gruppe der Formel 2 darstellt:

$R_1 =$ 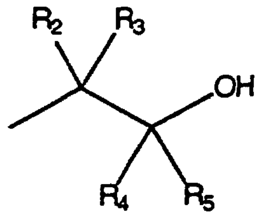 (Formel 2)

worin $R_2$, $R_3$, $R_4$, $R_5$ voneinander unabhängig sind und $C_1$-$C_4$-Alkyl, $C_1$-$C_4$-Hydroxyalkyl, oder Wasserstoff bedeuten.

2. Verfahren zur Oberfächennachvernetzung von Ausgangspolymeren, die Carboxylgruppen enthalten, charakterisiert durch die Verwendung eines Vernetzers der allgemeinen Formel 1 gemäß Anspruch 1.

3. Verfahren zur Vernetzung nach einem der Ansprüche 1 oder 2, in dem das Ausgangspolymer mit einem Vernetzer behandelt und während oder nach dem Behandeln durch Temperaturerhöhung nachvernetzt und getrocknet wird, wobei der Vernetzer in einem inerten Lösemittel enthalten ist.

4. Verfahren nach einem der Ansprüche 1 bis 3, wobei der Vernetzer N-Hydroxyethyl-2,3-Morpholindion ist.

5. Verfahren nach einem der Ansprüche 1 bis 4, wobei das Ausgangspolymere eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

6. Stoffgemisch enthaltend einen Vernetzer der Formel 1 gemäß Anspruch 1 oder Anspruch 4 und Lösemittel ausgewählt aus der Gruppe bestehend aus Wasser, ein Gemisch von Wasser mit einem oder mehreren in Wasser unbegrenzt löslichen organischen Lösemittel oder ein Gemisch von Wasser mit einem oder mehreren einfachen oder mehrfachfunktionellen Alkoholen.

7. Stoffgemisch nach Anspruch 6, **dadurch gekennzeichnet, dass** das Lösemittel ein Alkohol/Wasser-Gemisch mit einem Alkoholgehalt dieser Lösung von 10 - 90 Gew.-%, bevorzugt 15 - 65 Gew.-% ist.

8. Stoffgemisch nach einem der Ansprüche 6 oder 7, **dadurch gekennzeichnet, dass** der Alkohol Methanol, Ethanol, Isopropanol, Ethylenglykol, 1,2-Propandiol oder 1,3-Propandiol ist.

9. Stoffgemisch nach einem der Ansprüche 6 bis 8, **dadurch gekennzeichnet, dass** der Vernetzer im Stoffgemisch zu 0.1 bis 10 Gew.-%, insbesondere 0.5 bis 3 Gew.-% beträgt.

10. Verfahren nach einem der Ansprüche 1 bis 5, **dadurch gekennzeichnet, dass** ein Stoffgemisch nach einem der

Ansprüche 6 bis 9 verwendet wird.

**11.** Verfahren nach einem der Ansprüche 1 bis 5 oder 10, **dadurch gekennzeichnet, dass** das Stoffgemisch enthaltend Vernetzer und Lösemittel in einem Verhältnis von 0,1 - 20 Gew.-%, insbesondere 0,5 - 10 Gew.-% bezogen auf die Masse des Ausgangspolymeren eingesetzt wird.

**12.** Verfahren nach einem der Ansprüche 1 bis 5 oder 10 bis 11, **dadurch gekennzeichnet, dass** der Vernetzer in einer Dosierung von 0,01 - 5,0 Gew.%, bevorzugt 0,02 - 3,0 Gew.%, ganz besonders bevorzugt 0,03 - 1,0 Gew. %, insbesondere 0,05 - 0,1 Gew.% bezogen auf das Ausgangspolymer verwendet wird.

**13.** Polymer erhältlich nach einem Verfahren der Ansprüche 1 bis 5 oder 10 bis 12.

**14.** Verwendung der Polymere gemäss Anspruch 13 in Hygieneartikeln, Verpackungsmaterialien und in Nonwovens.

**15.** Verwendung von einem Stoffgemisch gemäß Anspruch 6 bis 9 zur Herstellung von Vernetzten oder durch Wärmebehandlung vernetzungsfähigen Polymeren.

## Claims

**1.** A process for crosslinking starting polymers for preparing hydrogel-forming polymers capable of absorbing aqueous fluids, which comprises using a crosslinker of the general formula 1:

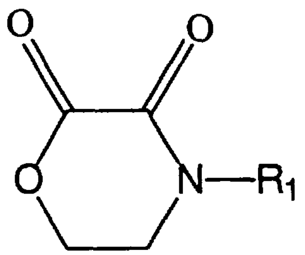

(Formula 1)

where $R_1$ is hydrogen, $C_1$-$C_4$-alkyl, $C_1$-$C_4$-hydroxyalkyl or a group of the formula 2:

$R_1 =$ 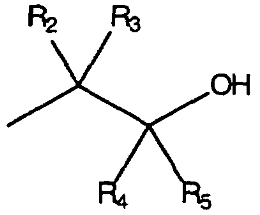

(Formula 2)

where $R_2$, $R_3$, $R_4$, and $R_5$ are each independently $C_1$-$C_4$-alkyl, $C_1$-$C_4$-hydroxyalkyl or hydrogen.

**2.** A process for surface postcrosslinking of starting polymers containing carboxyl groups, which comprises using a crosslinker of the general formula 1 as set forth in claim 1.

**3.** A process as claimed in either of claims 1 and 2, wherein the starting polymer is treated with a crosslinker and postcrosslinked and dried by raising the temperature during or after the treatment, the crosslinker being present in an inert solvent.

**4.** A process as claimed in any of claims 1 to 3, wherein the crosslinker is N-hydroxyethyl-2,3-morpholinedione.

**5.** A process as claimed in any of claims 1 to 4, wherein the starting polymer is a polymeric acrylic acid or a polyacrylate, especially a polymeric acrylic acid or a polyacrylate obtained by free-radical polymerization using a polyfunctional ethylenically unsaturated free-radical crosslinker.

**6.** A composition of matter comprising a crosslinker of the formula 1 as set forth in claim 1 or claim 4 and a solvent selected from the group consisting of water, a mixture of water with one or more organic solvents having unlimited solubility in water, and a mixture of water with one or more monohydric or polyhydric alcohols.

**7.** A composition of matter as claimed in claim 6, wherein the solvent is an alcohol-water mixture having an alcohol content of 10-90% by weight, preferably 15-65% by weight, based on this solution.

**8.** A composition of matter as claimed in either of claims 6 and 7, wherein the alcohol is methanol, ethanol, isopropanol, ethylene glycol, 1,2-propanediol or 1,3-propanediol.

**9.** A composition of matter as claimed in any of claims 6 to 8, wherein the crosslinker comprises from 0.1 to 10% by weight and especially from 0.5 to 3% by weight of the composition of matter.

**10.** A process as claimed in any of claims 1 to 5, wherein a composition of matter as claimed in any of claims 6 to 9 is used.

**11.** A process as claimed in any of claims 1 to 5 or 10, wherein the composition of matter comprising crosslinker and solvent is used in a ratio of 0.1-20% by weight and especially 0.5-10% by weight based on the mass of the starting polymer.

**12.** A process as claimed in any of claims 1 to 5 or 10 to 11, wherein the crosslinker is used in a dose of 0.01-5.0% by weight, preferably 0.02-3.0% by weight, more preferably 0.03-1.0% by weight and especially 0.05-0.1% by weight based on the starting polymer.

**13.** Polymer obtainable by a process as claimed in any of claims 1 to 5 or 10 to 12.

**14.** The use of the polymers of claim 13 in hygiene articles, packaging materials and nonwovens.

**15.** The use of a composition of matter as claimed in any of claims 6 to 9 for producing crosslinked polymers or polymers capable of being crosslinked by heating.

## Revendications

**1.** Procédé de réticulation de polymères de départ pour la préparation de polymères formateurs d'hydrogel absorbant des liquides aqueux, **caractérisé par** l'utilisation d'un agent de réticulation de la formule générale 1 :

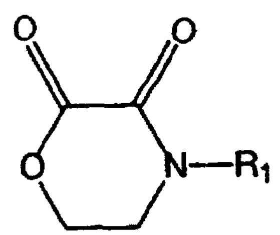

(Formule 1)

dans laquelle le radical $R_1$ représente soit de l'hydrogène, de l'alkyle en $C_1$-$C_4$, de l'hydroxyalkyle en $C_1$-$C_4$ ou un groupe de la formule 2 :

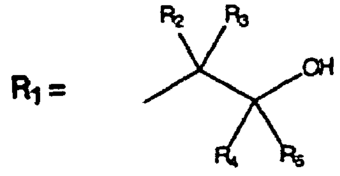

(Formule 2)

dans laquelle $R_2$, $R_3$, $R_4$, $R_5$ sont indépendants l'un de l'autre et représentent de l'alkyle en $C_1$-$C_4$, de l'hydroxyalkyle

en $C_1$-$C_4$ ou de l'hydrogène.

2. Procédé de postréticulation de surface de polymères de départ, qui contiennent des groupes carboxyle, **caractérisé par** l'utilisation d'un agent de réticulation de la formule générale 1 suivant la revendication 1.

3. Procédé de réticulation suivant l'une des revendications 1 et 2, dans lequel le polymère de départ est traité avec un agent de réticulation et postréticulé et séché pendant ou après le traitement par élévation de température, l'agent de réticulation étant contenu dans un solvant inerte.

4. Procédé suivant l'une des revendications 1 à 3, dans lequel l'agent de réticulation est de la N-hydroxyéthyl-2,3-morpholinedione.

5. Procédé suivant l'une des revendications 1 à 4, dans lequel le polymère de départ est un acide acrylique polymère ou un polyacrylate, en particulier un acide acrylique polymère ou un polyacrylate qui a été obtenu par polymérisation radicalaire et pour lequel un agent de réticulation radicalaire éthyléniquement insaturé plurifonctionnel a été utilisé.

6. Mélange contenant un agent de réticulation de la formule 1 suivant la revendication 1 ou la revendication 4 et un solvant sélectionné parmi le groupe constitué de l'eau, d'un mélange d'eau avec un ou plusieurs solvants organiques solubles de manière illimitée dans l'eau ou d'un mélange d'eau avec un ou plusieurs alcools à une ou plusieurs fonctions.

7. Mélange suivant la revendication 6, **caractérisé en ce que** le solvant est un mélange d'alcool/eau présentant une teneur en alcool de cette solution de 10-90% en poids, de préférence de 15-65% en poids.

8. Mélange suivant l'une des revendications 6 et 7, **caractérisé en ce que** l'alcool est du méthanol, de l'éthanol, de l'isopropanol, de l'éthylèneglycol, du 1,2-propanediol ou du 1,3-propanediol.

9. Mélange suivant l'une des revendications 6 à 8, **caractérisé en ce que** l'agent de réticulation se trouve dans le mélange à 0,1 jusqu'à 10% en poids, en particulier à 0,5 jusqu'à 3% en poids.

10. Procédé suivant l'une des revendications 1 à 5, **caractérisé en ce qu'**on utilise un mélange suivant l'une des revendications 6 à 9.

11. Procédé suivant l'une des revendications 1 à 5 ou 10, **caractérisé en ce qu'**on met en oeuvre le mélange contenant de l'agent de réticulation et du solvant dans une proportion de 0,1-20% en poids, en particulier de 0,5-10% en poids, par rapport à la masse du polymère de départ.

12. Procédé suivant l'une des revendications 1 à 5 ou 10 et 11, **caractérisé en ce que** l'agent de réticulation est utilisé en un dosage de 0,01-5,0% en poids, de préférence de 0,02-3,0% en poids, tout particulièrement de 0,03-1,0% en poids, en particulier de 0,05-0,1% en poids, par rapport au polymère de départ.

13. Polymère que l'on peut obtenir suivant un procédé suivant l'une des revendications 1 à 5 ou 10 à 12.

14. Utilisation des polymères suivant la revendication 13, dans des articles d'hygiène, des matières d'emballage et des non-tissés.

15. Utilisation d'un mélange suivant l'une des revendications 6 à 9, pour la préparation de polymères réticulés ou susceptibles de réticuler par traitement thermique.